(19) Europäisches Patentamt
European Patent Office
Office européen des brevets

(11) Veröffentlichungsnummer: **0 438 811 A2**

(12) **EUROPÄISCHE PATENTANMELDUNG**

(21) Anmeldenummer: **90125838.4**

(22) Anmeldetag: **31.12.90**

(51) Int. Cl.5: **C07D 239/34**, C07D 239/26, C07D 213/30, C07D 237/08, C07D 285/12, C07C 69/63, C07C 255/19, C09K 19/34, C09K 19/12, C09K 19/20

(30) Priorität: **24.01.90 DE 4001965**

(43) Veröffentlichungstag der Anmeldung: **31.07.91 Patentblatt 91/31**

(84) Benannte Vertragsstaaten: **CH DE FR GB LI NL**

(71) Anmelder: **HOECHST AKTIENGESELLSCHAFT**
**Postfach 80 03 20**
**W-6230 Frankfurt am Main 80(DE)**

(72) Erfinder: **Scherowsky, Günter, Prof. Dr.**
**Winklerstrasse 18b**
**W-1000 Berlin(DE)**
Erfinder: **Gay, Jürgen**
**Bismarckstrasse 51a**
**W-1000 Berlin(DE)**
Erfinder: **Escher, Claus, Dr.**
**Amselweg 3**
**W-6109 Mühltal(DE)**
Erfinder: **Wingen, Rainer, Dr.**
**Brunnenstrasse 1**
**W-6234 Hattersheim am Main(DE)**

(54) **Ester von 3-Halogen- bzw. 3-Cyano-substituierten 2-Methylalkancarbonsäuren und ihre Verwendung als Dotierstoffe in flüssigkristallinen Mischungen.**

(57) Ester aus in 3-Position substituierten 2-Methylalkancarbonsäuren und mesogenen Alkoholen gemäß der allgemeinen Formel (I)

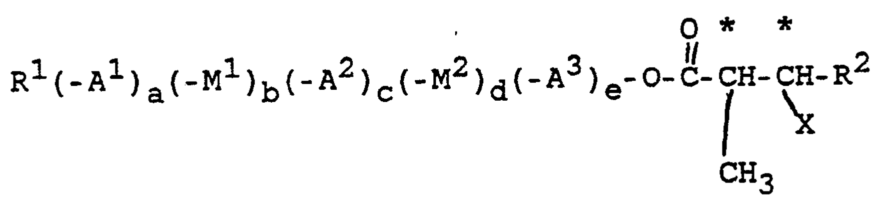

in der bedeuten:

| | |
|---|---|
| $R^1$ | z.B. geradkettiges oder verzweigtes (mit oder ohne) asymmetrisches C-Atom) Alkyl mit 1 bis 14 C-Atomen, |
| $A^1$, $A^2$, $A^3$ | gleich oder verschieden z.B. 1,4-Phenylen, bei dem ein oder zwei H-Atome durch F, Cl und/oder CN ersetzt sein können, trans-1,4-Cyclohexylen oder Pyrimidin-2,5-diyl |
| $M^1$, $M^2$ | gleich oder verschieden CO-O, O-CO, $CH_2$-O, O-$CH_2$, C≡C, CH=CH oder $CH_2$-$CH_2$, |
| X | F, Cl oder CN |
| $R^2$ | geradkettiges oder verzweigtes Alkyl mit 1 bis 10 C-Atomen oder Cycloalkyl mit 5 bis 8 C-Atomen, und |
| a | gleich 1, und |
| b, c, d, e | gleich Null oder 1 mit der Maßgabe, daß c+e gleich 1 oder 2 ist. |

zeichnen sich durch eine hohe Stabilität ihrer Mesophasen gegenüber UV-Bestrahlung und Erwärmung aus. Sie

EP 0 438 811 A2

Xerox Copy Centre

lassen sich vorteilhaft als Dotierstoffe in flüssigkristallinen Mischungen einsetzen.

# ESTER VON 3-HALOGEN- BZW. 3-CYANO-SUBSTITUIERTEN 2-METHYLALKANCARBONSÄUREN UND IHRE VERWENDUNG ALS DOTIERSTOFFE IN FLÜSSIGKRISTALLINEN MISCHUNGEN

Die ungewöhnliche Kombination von anisotropem und fluidem Verhalten der Flüssigkristalle hat zu ihrer Verwendung in einer Vielzahl verschiedener elektro-optischer Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelbrechung, der Einlagerung dichroitisch absorbierender Farbstoffmoleküle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall ("liquid crystal")-Mischungen und somit auch an einer Vielzahl mesogener Verbindungen unterschiedlicher Struktur. Dies gilt sowohl für die Gebiete, bei denen nematische LC-Phasen verwendet werden, als auch für solche mit smektischen LC-Phasen.

Viele der für LC-Mischungen geeigneten Verbindungen lassen sich durch ein Aufbauprinzip beschreiben [siehe z.B. J. Am. Chem. Soc. 108, 4736 (1986), Struktur I; Science 231, 350 (1986), Fig. 1 A; J. Am. Chem. Soc. 108, 5210 (1986), Fig. 3], bei dem Kerne aus cyclischen Verbindungen - Aromaten, Heteroaromaten, aber auch gesättigte Ringsysteme - mit geradkettigen oder in der Kette durch kleine Gruppen (z.B. Methyl, Chlor) substituierten und somit verzweigten Alkylseitenketten verknüpft sind.

Auf besonderes Interesse sind in den letzen Jahren ferroelektrische flüssigkristalline Mischungen (FLC-Mischungen) gestoßen (siehe z.B. Lagerwall et al. "Ferroelectric Liquid Crystals for Displays", SID Symposium, October Meeting 1985, San Diego, Ca. USA). Für die praktische Verwendung von ferroelektrischen Flüssigkristallen in elektro-optischen Anzeigen werden chirale, geneigt-smektische Phasen wie z.B. $S_c$-Phasen benötigt [siehe R.B. Meyer, L. Liébert, L. Strzelecki und P. Keller, J. Physique 36, L-69 (1975)], die über einen großen Temperaturbereich stabil sind. Dieses Ziel kann man mit Verbindungen erreichen, die selbst solche Phasen, z.B. $S_c$-Phasen, ausbilden oder aber, indem man nicht chirale, geneigt-smektische Phasen ausbildende Verbindungen mit optisch aktiven Verbindungen dotiert [siehe M. Brunet, Cl. Williams, Ann. Phys. 3, 237 (1978)].

Für die praktische Anwendung chiraler Dotierstoffe in flüssigkristallinen Mischungen sind folgende Kenngrößen besonders wichtig:

a) Beeinflussung der Phasenbreite der Gesamtmischung,
b) Pitch in der $N^*$- bzw. der $S_C^*$ -Phase
c) spontane Polarisation,
d) Rotationsviskosität,
e) Stabilität der Mesophasen sowie
f) Mischbarkeit mit anderen Komponenten.

In der Literatur wird eine Vielzahl von unterschiedlichen chiralen Verbindungen beschrieben, die als Dotierstoffe für flüssigkristalline Mischungen geeignet sind und die für die genannten Kenngrößen mehr oder weniger gute Werte aufweisen.

So sind als Dotierstoffe für LC-Mischungen geeignete Verbindungen, die in der Seitenkette ein asymmetrisches C-Atom enthalten, an das als Substituent eine Halogen- oder eine Cyanogruppe gebunden ist, ganz allgemein aus folgenden Patentanmeldungen oder Patentschriften bekannt: DE 33 32 992, EP 237 007, EP 267 585, EP 315 958, JP 63-119 449, US 4.777.280, US 4.695.650, WO 86/00087, WO 86/06373 und WO 87/07890.

Derivate von 2-Cyano-2-alkyl-alkancarbonsäuren sowie deren Verwendung in LC-Mischungen werden in EP 315 996, EP 316 011 und WO 88/03530 beschrieben.

Ester von 2-Halogen-alkancarbonsäuren sind aus EP 159 872, EP 174 816, EP 207 712, EP 242 716, EP 285 141, DE 35 15 373, DE 37 30 859 und DD 240 385 bereits bekannt. Bei diesen Verbindungen kann auch das C(3)-Atom ein asymmetrisches C-Atom sein.

Verbindungen, die Ester von 3-Chlor-alkansäuren darstellen, sind aus DD 257 638 bekannt. Jedoch können nach der dort angegebenen allgemeinen Formel C(2) und C(3) nicht beide zugleich asymmetrische C-Atome darstellen.

Verbindungen mit zwei asymmetrischen C-Atomen und deren Verwendung in flüssigkristallinen Mischungen werden in WO 87/05018 beschrieben, die asymmetrischen C-Atome sind jedoch durch ein mesogenes Gerüst voneinander getrennt.

Der vorliegenden Erfindung liegt die Aufgabe zugrunde, neue Dotierstoffe für flüssigkristalline Mischungen bereitzustellen, die in verschiedenen LC-Mischungen eingesetzt werden können und die sich sowohl durch eine hohe thermische Mesophasenstabilität wie auch durch eine große Beständigkeit der Mesophasen gegenüber UV-Bestrahlung auszeichnen.

Als Maß für die Stabilität der Mesophasen der Verbindungen wird hierbei die Veränderung der Mesophasenübergangstemperaturen nach Erwärmung bzw. UV-Bestrahlung angenommen.

Die Aufgabe wird durch die nachfolgend beschriebenen Verbindungen gelöst.

Gegenstand der Erfindung sind Ester aus in 3-Position substituierten 2-Methylalkancarbonsäuren und mesogenen Alkoholen gemäß der allgemeinen Formel (I)

$$R^1\ (-A^1)_a\ (-M^1)_b\ (-A^2)_c\ (-M^2)_d\ (-A^3)_e\text{-O-}\overset{\overset{\displaystyle O}{\|}}{C}\text{-}\underset{\underset{\displaystyle CH_3}{|}}{\overset{*}{C}H}\text{-}\underset{\underset{\displaystyle X}{|}}{\overset{*}{C}H}\text{-}R^2$$

in der bedeuten:

$R^1$

geradkettiges oder verzweigtes (mit oder ohne asymmetrisches C-Atom) Alkyl mit 1 bis 14 C-Atomen, wobei auch einer oder zwei nicht benachbarte $-CH_2$-Gruppen durch -O-, -CO-, -CO-O-, -O-CO-, -CH=CH-, -C≡C-,

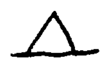

oder $-Si(CH_3)_2-$ ersetzt und wobei auch ein oder mehrere H-Atome des Alkylrestes durch F, Cl oder CN substituiert sein können,

$A^1$, $A^2$, $A^3$

gleich oder verschieden 1,4-Phenylen, bei dem ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, Pyrazin-2,5-diyl, Pyridazin-3,6-diyl, Pyridin-2,5-diyl, Pyrimidin-2,5-diyl, (1,3,4)-Thiadiazol-2,5-diyl, 1,3-Dioxan-2,5-diyl oder Naphthalin-2,6-diyl,

$M^1$, $M^2$

gleich oder verschieden CO-O, O-CO, $CH_2$-O, O-$CH_2$, C≡C, CH=CH oder $CH_2$-$CH_2$,

X

F, Cl oder CN

$R^2$

geradkettiges oder verzweigtes Alkyl mit 1 bis 10 C-Atomen oder Cycloalkyl mit 5 bis 8 C-Atomen, und

a

gleich 1

b, c, d, e

gleich Null oder 1 mit der Maßgabe, daß c + e gleich 1 oder 2 ist.

Bevorzugt werden solche Ester, bei denen

$(-A^1)_a\ (-M^1)_b\ (-A^2)_c\ (-M^2)_d\ (-A^3)_e$- für folgende Gruppen steht:

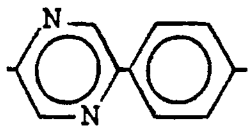 , 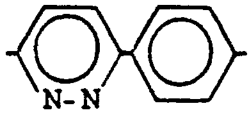 , 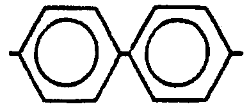 ,

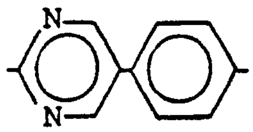 , 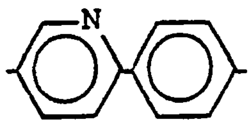 , 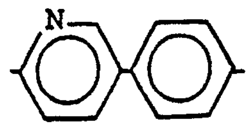

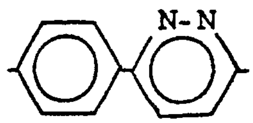 , 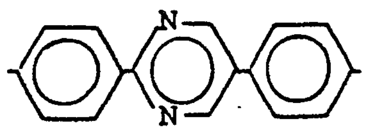 ,

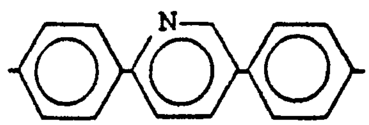 , 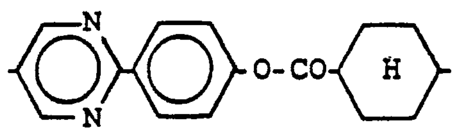 ,

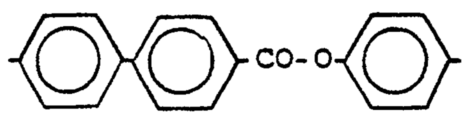 , 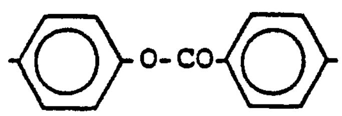 ,

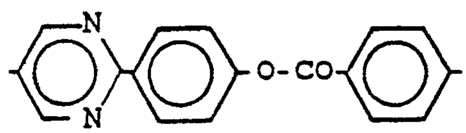 , 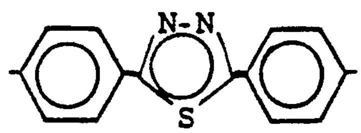 ,

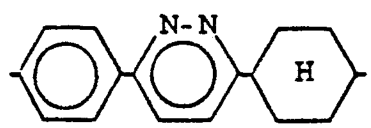 , 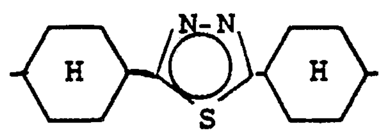 ,

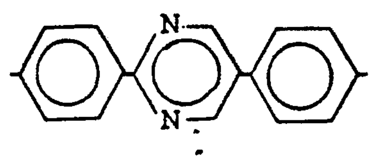 , 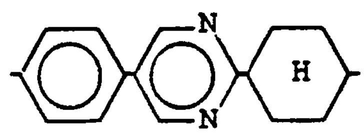 .

Besonders bevorzugt werden Ester, bei denen

$R^1$

geradkettiges oder verzweigtes Alkyl mit 1 bis 14 C-Atomen bedeutet, wobei auch eine oder zwei $-CH_2-$ Gruppen durch -O-, -CO-, -O-CO- oder -CO-O- ersetzt sein können, und

$(-A^1)_a$ $(-M^1)_b$ $(-A^2)_c$ $(-M^2)_d$ $(-A^3)_e$- für eine der folgenden Gruppierungen steht:

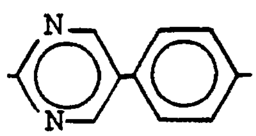 , 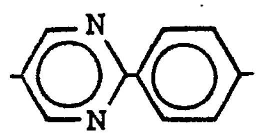 , 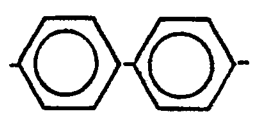 ,

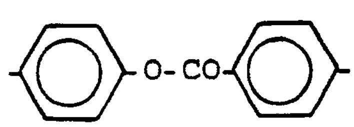 .

Ebenfalls besonders bevorzugt werden Ester, bei denen mindestens einer der Reste $A^1$, $A^2$, $A^3$ ein 1,4-Phenylen und ein weiterer Pyridin-2,5-diyl bedeutet.

Ein wesentlicher Vorteil der Ester der allgemeinen Formel (I) gegenüber den im Stand der Technik

bekannten α-Halogen substituierten Carbonsäureestern ist die Mesophasen-Stabilität. Die beschriebenen 2-Methyl-3-halogen- bzw. 2-Methyl-3-cyano-substituierten Carbonsäureester sind nicht nur in verschiedenen LC-Mischungen verwendbar und gut mischbar mit anderen Komponenten, sondern zeigen überraschenderweise auch eine hohe Beständigkeit ihrer Mesophasen gegenüber Erwärmung und UV-Bestrahlung, was für ihre praktische Anwendung in elektrooptischen Schalt- und Anzeigeelementen von großem Vorteil ist.

Die genannten Ester der allgemeinen Formel (I) lassen sich durch Umsetzung einer mesogenen Verbindung der allgemeinen Formel (II) mit einem monofunktionellen Carbonsäurederivat der allgemeinen Formel (III)

$R^1 (-A^1)_a (-M^1)_b (-A^2)_c (-M^2)_d (-A^3)_e\text{-}Z$ (II)

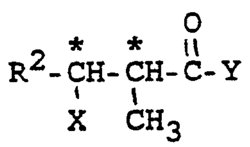

(III)

synthetisieren, wobei Z gleich OH oder O-Alkali bedeutet und Y für einen, bei der Reaktion abgehenden Substituenten wie OH oder Halogen steht. Darüber hinaus ist auch die Herstellung der erfindungsgemäßen Ester ausgehend von gängigen Carbonsäurederivaten wie Anhydriden oder einfachen Estern möglich.
Die als Edukte dienenden Verbindungen (II) und (III) sind nach literaturbekannten Verfahren darstellbar.

Die Ester der allgemeinen Formel (I) sind als Dotierstoffe für flüssigkristalline Mischungen aus den genannten Gründen besonders geeignet, wobei unter dem Begriff "flüssigkristalline Mischungen" solche mit cholesterischen, orthogonal smektischen oder geneigt ("tilted")-smektischen, insbesondere mit ferroelektrischen $S_C^*$ -Phasen zu verstehen sind. Die Flüssigkristallmischungen bestehen aus 2 bis 20, vorzugsweise 2 bis 15 Komponenten, darunter als eine Komponente mindestens einen der erfindungsgemäß beanspruchten chiralen Ester der allgemeinen Formel (I).

Die anderen Bestandteile werden vorzugsweise ausgewählt aus den bekannten Verbindungen mit nematischen, cholesterischen und/oder smektischen, z. B. $S_A$-Phasen, und/oder geneigt smektischen Phasen; dazu gehören beispielsweise
Biphenyle, Terphenyle, Phenylcyclohexane, Pyrimidine, Zimtsäureester, Cholesterinester, verschiedene überbrückte, terminal-polar mehrkernige Ester von p-Alkylbenzoesäuren. Im allgemeinen liegen die im Handel erhältlichen Flüssigkristallmischungen bereits vor der bereits vor der Zugabe der optisch aktiven Verbindung(en) als Gemische verschiedener Komponenten vor, von denen mindestens eine mesogen ist, d. h. als Verbindung in derivatisierter Form oder im Gemisch mit anderen Komponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Von der oder den erfindungsgemäßen Verbindung(en) enthalten die Flüssigkristallmischungen im allgemeinen 0,01 bis 20 Mol-%, insbesondere 0,1 bis 10 Mol-%.

Solche, mit Verbindungen der allgemeinen Formel (I) dotierte flüssigkristalline Mischungen sind besonders für die Verwendung in elektrooptischen Schalt- und Anzeigevorrichtungen (Displays) geeignet, da für eine breite Verwendbarkeit solcher Displays eine hohe thermische Stabilität und eine UV-Beständigkeit der Mesophasen des flüssigkristallinen Materials notwendig ist. Schalt- und Anzeigevorrichtungen (LC-Displays) weisen u. a. folgende Bestandteile auf: ein flüssigkristallines Medium, Trägerplatten (z. B. aus Glas oder Kunststoff), beschichtet mit transparenten Elektroden, mindestens eine Orientierungsschicht, Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie z. B. Dünnschichttransistoren (TFT) und Metall-Isolator-Metall-(MIM)-Elemente. Im Detail ist der Aufbau von Flüssigkristalldisplays bereits in einschlägigen Monographien beschrieben (z. B. E. Kaneko, "Liquid Crystal TV Displays: Principles and Applications of Liquid Crystal Displays", KTK Scientific Publishers, 1987, Seiten 12 - 30 und 163 - 172). Die Erfindung wird durch die nachfolgenden Beispiele verdeutlicht.

**Beispiele**

Allgemeine Vorschrift für die Synthese der Ester von 3-Halogen-2-methylalkancarbonsäuren

Die Synthese der 3-Halogen- bzw. 3-Cyano-2-methyl-alkansäuren - sowohl für optisch aktive als auch für racemische Verbindungen - erfolgte wie im nachfolgenden Schema angegeben:

Schema 1:

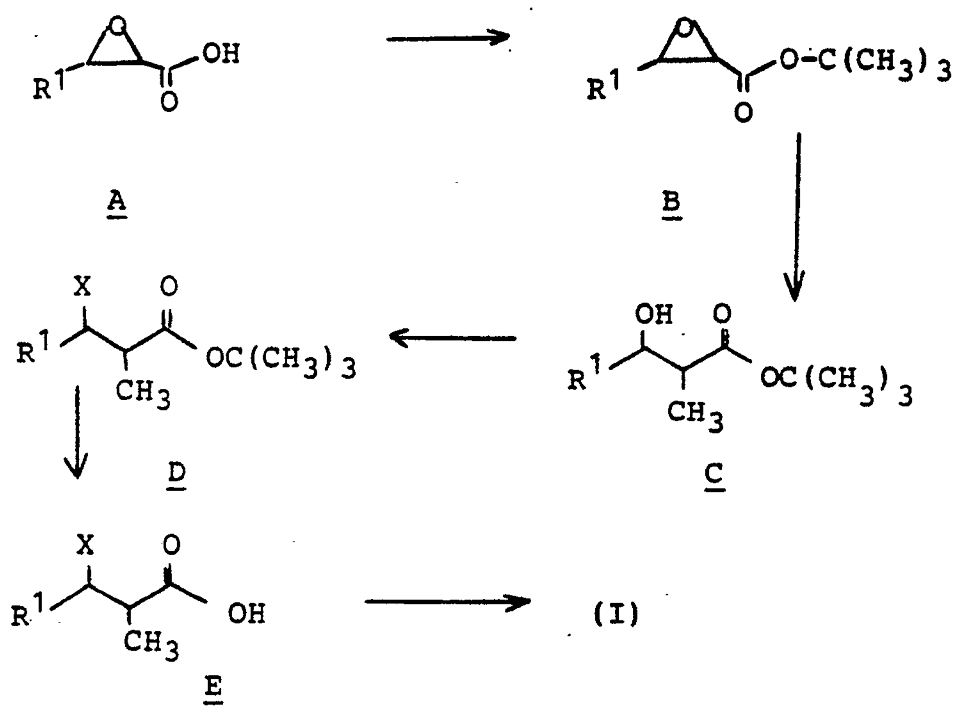

A B C D E (I)

Oxiran-2-carbonsäuren A (hergestellt wie in EP-A-0 292 954 beschrieben) wurden in die entsprechenden tert-Butylester B überführt. Die Ringöffnung mit metallorganischen Reagenzien, z.B. Dialkylcupraten, ergab die 3-Hydroxy-2-methyl-ester C. Die Hydroxyfunktion wurde unter schonenden Bedingungen, z.B. mit $PCl_5/CaCO_3$ oder mit Diethylaminoschwefeltrifluorid, in die Halogenverbindung D - oder nach Überführung in die entsprechenden Tosylate oder Mesylate - mit (Alkali-)Cyaniden in die Cyanoverbindung D überführt.

Nach Abspaltung der tert-Butylgruppe, z.B. mit Trifluoressigsäure, wurde die 3-Halogen- bzw. 3-Cyano-2-methyl-alkancarbonsäure E erhalten.

**Beispiel 1**

Eine Lösung von 1.15 mmol N,N'-Dicyclohexylcarbodiimid und 0.12 mmol 4-Dimethylamino-pyridin in 10 ml Dichlormethan wurde mit 1.15 mmol (2S,3R)-3-Chlor-2-methyl-undecansäure E und 1.15 mmol 4-(2-Octyloxy-pyrimidin-5-yl)phenol versetzt. Nach Reaktionsende wurde der Dicyclohexylharnstoff abfiltriert und das Filtrat chromatographisch gereinigt. Man erhielt (2S,3R)-3-Chlor-2-methyl-undecansäure-4-(2-octyloxy-pyrimidin-5-yl)phenyl-ester. Die Verbindung wies folgende Phasenfolge auf: K 41.5 (36.5 $S_A$)I

(2S, 3R) 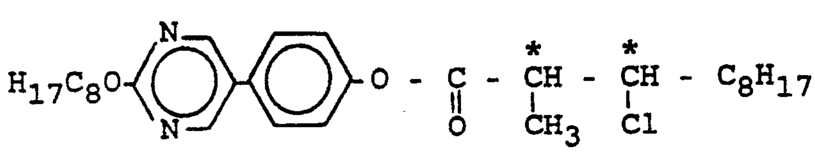

Analog lassen sich darstellen:

(2S,3R)-3-Chlor-2-methyl-hexansäure-4-(2-hexyloxy-pyrimidin-5-yl)phenyl-ester
(2S,3R)-3-Chlor-2-methyl-heptansäure-4-(2-nonyl-pyrimidin-5-yl)phenyl-ester
(2S,3R)-3-Chlor-2-methyl-pentansäure-4-(5-octyloxy-pyrimidin-2-yl)phenyl-ester
(2S,3R)-3-Chlor-2-methyl-octansäure-<2-(4-octyloxyphenyl)pyrimidin-5-yl>ester
(2S,3R)-3-Chlor-2-methyl-decansäure-4-(5-octyl-pyridin-2-yl)phenyl-ester
(2S,3R)-3-Chlor-2-methyl-nonansäure-5-(5-nonyl-pyrazin-2-yl)phenyl-ester
(2S,3R)-3-Chlor-2-methyl-nonansäure-4-(5-undecyl-1,3,4-thiadiazol-2-yl)phenyl-ester
(2R,3S)-3-Chlor-2-methyl-heptansäure-4-(2,3-difluor-4-heptyloxy-phenyl)phenyl-ester

(2R,3S)-3-Chlor-2-methyl-hexansäure-4-(4-decycloxybenzoyl-oxy)phenyl-ester
(2S,3R)-3-Chlor-2-methyl-decansäure-4-<2,3-difluor-4-hexyloxy-biphenyl-4-'-carbonyloxy>phenyl-ester
(2S,3R)-3-Chlor-2-methyl-heptansäure-4-<4-(5-nonyl-pyrimidin-2-yl)phenyl-oxycarbonyl>cyclohexyl-ester
(2S,3R)-3-Fluor-2-methyl-hexansäure-4-(2-octyloxy-pyrimidin-5-yl)phenyl-ester
(2S,3R)-3-Fluor-2-methyl-pentansäure-4-(5-heptyloxy-pyrimidin-2-yl)phenyl-ester
(2S,3R)-3-Fluor-2-methyl-heptansäure-4-(5-decyl-pyrimidin-2-yl)phenyl-ester
(2S,3R)-3-Fluor-2-methyl-octansäure-<2-(4-octyloxyphenyl)pyrimidin-5-yl>ester
(2S,3R)-3-Fluor-2-methyl-heptansäure-4-(5-octyl-pyridin-2-yl)phenyl-ester
(25,3R)-3-Fluor-2-methyl-octansäure-4-(6-octyl-pyridazin-3-yl)phenyl-ester
(2S,3R)-3-Fluor-2-methyl-pentansäure-4-(5-heptyl-1,3,4-thiadiazol-2-yl)phenyl-ester
(2R,3S)-3-Fluor-2-methyl-hexansäure-4-(2,3-difluor-4-heptyloxy-phenyl)phenyl-ester
(2R,3S)-3-Fluor-2-methyl-hexansäure-4-(4-decycloxybenzoyl-oxy)phenyl-ester
(2S,3R)-3-cyano-2-methyl-hexansäure-4-(2-hexyloxy-pyrimidin-5-yl)phenyl-ester
(2S,3R)-3-Cyano-2-methyl-heptansäure-4-(5-octyl-pyrimidin-2-yl)phenyl-ester
(2S,3R)-3-Cyano-2-methyl-hexansäure-4-(5-heptyl-1,3,4-thiadiazol-2-yl)Phenyl-ester
(2S,3R)-3-Cyano-2-methyl-pentansäure-4-(5-octyl-pyridin-2-yl)phenyl-ester

**Anwendungsbeispiele**

Die erfindungsgemäße Verbindung (2S,3R)-3-Chlor-2-methyl-undecansäure-4-(2-octyloxy-pyrimidin-5-yl)phenyl-ester (Beispiel 1) wurde zu 10 Mol-% in einer nicht-chiralen flüssigkristallinen Wirtsmatrix (W), die die Phasenfolge $S_c$ 84.5 $S_A$ 92.5 N 104.5 I aufweist, gelöst.

Dabei resultierte folgende Phasenfolge der Mischung

$S_c^*$ 78 $S_A^*$ 84.5 N* 94 I.

Die erfindungsgemäße Mischung M wies bei einer Temperatur von 25 °C eine spontane Polarisation von 8 $nC/cm^2$ auf.

Im Vergleich zu den α-substituierten Chlorcarbonsäureestern weisen die erfindungsgemäßen Verbindungen gegenüber hohen Temperaturen und UV-Bestrahlung eine größere Stabilität der Mesophasen auf (gemessen als Depression der Temperaturen der Mesophasenübergänge).

Nach 5 Stunden bei einer Temperatur von 150 °C ergab sich folgende Depression der Mesophasenübergangstemperaturen:

$$S_C^*/S_A^*: 0\ K; \quad S_C^*/N^*: 0\ K; \quad N^*/I: 0\ K$$

Nach 5-stündiger UV-Bestrahlung (Wellenlänge größer als 380 nm) ergab sich folgende Depression der Mesophasenübergangstemperaturen:

$S_c^*$ /$S_A^*$ : 1,5 K; $S_A$/N*: 1,5 K;
N*/I: 1 K.

Für die α-substituierte Vergleichssubstanz (2S,3R)-2-Chlor-3-methyl-pentansäure-4-(2-nonyloxy-pyrimidin-5-yl)phenyl-ester (V) ergab sich nach 5 h bei einer Temperatur von 150 °C folgende Depression der Mesophasenübergangstemperaturen:

$S_c^*$ /$S_A^*$ : 2 K; $S_A^*$ /N*: 1 K; N*/I: 1 K;

nach 5-stündiger UV-Bestrahlung: $S_c^*$ /$S_A^*$ : 8 K; $S_A$/N*: 4 K; N*/I: 10 K

Besonders wichtig ist die im Vergleich zur Vergleichsverbindung V hohe Verdrillungskraft (HTP) der erfindungsgemäßen Verbindung in cholesterischen Phasen. Die Verdrillungskraft in der cholesterischen Phase wurde nach der Methode von Granjan-Cano bestimmt (siehe: R. Cano Bull. Soc. Franc. Miner. Crist. 90, 333, (1967)).

Während V bei einer Temperatur von 25° in der Wirtsmatrix W eine HTP von kleiner als 1 $\mu m^{-1}$ aufwies, zeigte die Verbindung X eine HTP von 6.6 $\mu m^{-1}$ (Vorzeichen jeweils negativ). Deshalb ließ sich X im Gegensatz zu V vorteilhaft zur Helixkompensation in der cholesterischen Phase einsetzen.

## Patentansprüche

1. Ester aus in 3-Position substituierten 2-Methylalkancarbonsäuren und mesogenen Alkoholen gemäß der allgemeinen Formel (I)

$$R^1\ (-A^1)_a\ (-M^1)_b\ (-A^2)_c\ (-M^2)_d\ (-A^3)_e\text{-O-}\overset{O}{\overset{\|}{C}}\text{-}\underset{CH_3}{\overset{*}{C}H}\text{-}\underset{X}{\overset{*}{C}H}\text{-}R^2$$

in der bedeuten:

$R^1$

geradkettiges oder verzweigtes (mit oder ohne asymmetrisches C-Atom) Alkyl mit 1 bis 14 C-Atomen, wobei auch einer oder zwei nicht benachbarte $-CH_2$-Gruppen durch -O-, -CO-, -CO-O-, -O-CO-, -CH = CH-, -C≡C-,

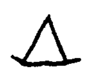

oder $-Si(CH_3)_2$- ersetzt und wobei auch ein oder mehrere H-Atome des Alkylrestes durch F, Cl oder CN substituiert sein können,

$A^1$ $A^2$ $A^3$

gleich oder verschieden 1,4-Phenylen, bei dem ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, Pyrazin-2,5-diyl, Pyridazin-3,6-diyl, Pyridin-2,5-diyl, Pyrimidin-2,5-diyl, (1,3,4)-Thiadiazol-2,5-diyl, 1,3-Dioxan-2,5-diyl oder Naphthalin-2,6-diyl,

$M^1$ $M^2$

gleich oder verschieden CO-O, O-CO, $CH_2$-O, O-$CH_2$, C≡C, CH = CH oder $CH_2$-$CH_2$,

X

F, Cl oder CN

$R^2$

geradkettiges oder verzweigtes Alkyl mit 1 bis 10 C-Atomen oder Cycloalkyl mit 5 bis 8 C-Atomen, und

a

gleich 1, und

b, c, d, e

gleich Null oder 1 mit der Maßgabe, daß c + e gleich 1 oder 2 ist.

2. Ester nach Anspruch 1, dadurch gekennzeichnet, daß
$(-A^1)_a$ $(-M^1)_b$ $(-A^2)_c$ $(-M^2)_d$ $(-A^3)_e$- bedeutet:

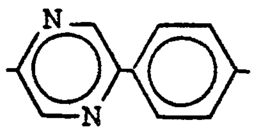 , 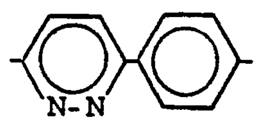 , 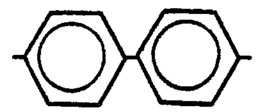 ,

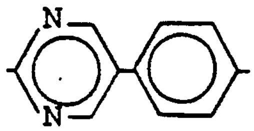 , 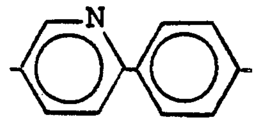 , 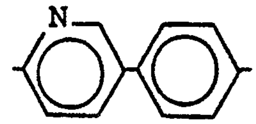

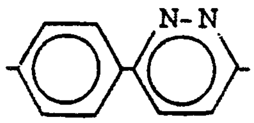 , 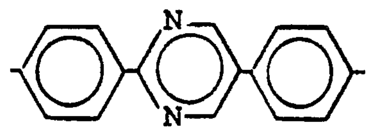 ,

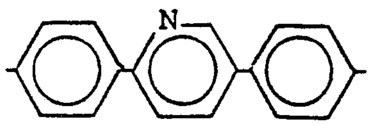 , 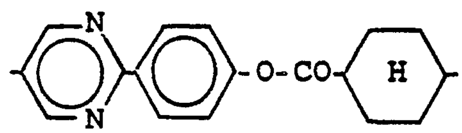 ,

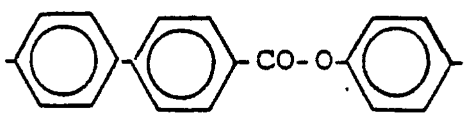 , 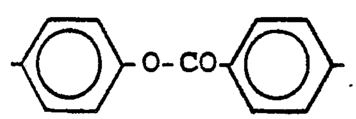 ,

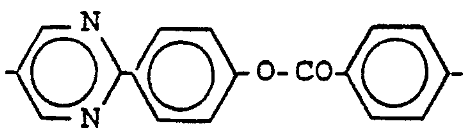 , 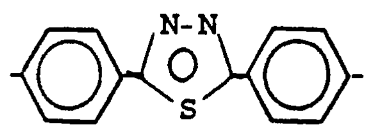 ,

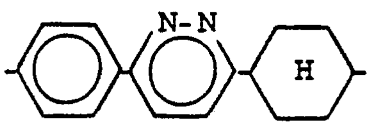 , 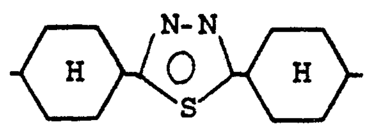 ,

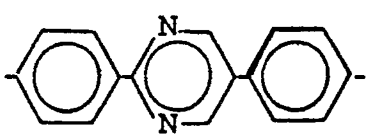 , 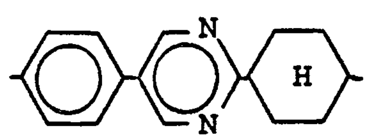 .

3. Ester nach Anspruch 1, dadurch gekennzeichnet, daß
$R^1$
geradkettiges oder verzweigtes Alkyl mit 1 bis 14 C-Atomen bedeutet, wobei auch eine oder zwei $-CH_2-$ Gruppen durch -O-, -CO-, -O-CO- oder -CO-O- ersetzt sein können, und $(-A^1)_a$ $(-M^1)_b$ $(-A^2)_c$ $(-M^2)_d$ $(-A^3)_e$- für folgende Gruppierung steht:

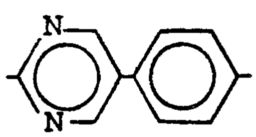 , 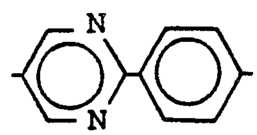 , 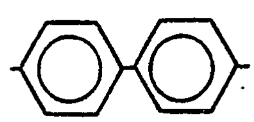 ,

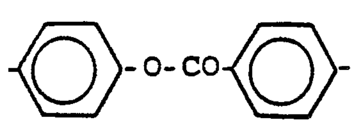 .

4. Ester nach Anspruch 2, dadurch gekennzeichnet, daß mindestens einer der Reste $A^1$, $A^2$, $A^3$ 1,4-Phenylen und ein weiterer Pyridin-2,5-diyl bedeuten.

5. Verfahren zur Herstellung eines Esters der allgemeinen Formel (I) nach Anspruch 1, dadurch gekennzeichnet, daß eine mesogene Verbindung der allgemeinen Formel (II) mit einem monofunktionellen Carbonsäurederivat der allgemeinen Formel (III)

$R^1 (-A^1)_a (-M^1)_b (-A^2)_c (-M^2)_d (-A^3)_e\text{-}Z$ (II)

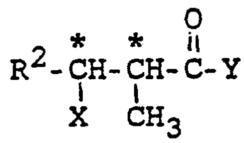

(III)

umgesetzt wird, wobei Z gleich OH oder O-Alkali bedeutet, und Y für einen bei der Reaktion abgehenden Substituenten wie OH oder Halogen steht.

6. Verwendung eines Esters der allgemeinen Formel (I) nach Anspruch 1 als Komponente in flüssigkristallinen Mischungen.

7. Verwendung gemäß Anspruch 6, dadurch gekennzeichnet, daß die flüssigkristalline Mischung smektisch ist.

8. Verwendung gemäß Anspruch 6, dadurch gekennzeichnet, daß die smektisch flüssigkristalline Mischung ferroelektrisch ist.

9. Flüssigkristalline Mischung bestehend aus 2 bis 20 Komponenten, dadurch gekennzeichnet, daß sie als eine Komponente mindestens einen Ester der allgemeinen Formel nach Anspruch 1 enthält.

10. Flüssigkristalline Mischung gemäß Anspruch 9, dadurch gekennzeichnet, daß sie 0,01 bis 20 Mol-% des Esters (I) enthält.

11. Flüssigkristalline Mischung gemäß Anspruch 9, dadurch gekennzeichnet, daß sie 0,1 bis 10 Mol-% des Esters (I) enthält.

12. Elektrooptische Schalt- und Anzeigevorrichtung enthaltend ein flüssigkristllines Medium, zwei Elektroden, zwei Trägerplatten sowie mindestens eine Orientierungsschicht, dadurch gekennzeichnet, daß das flüssigkristalline Medium eine flüssigkristalline Mischung gemäß Anspruch 9 ist.